# EUROPEAN PATENT APPLICATION

(11) **EP 4 239 082 A1**
(43) Date of publication of application: **06.09.2023**
(21) Application number: 22305244.0
(22) Date of filing: 02.03.2022
(51) Int. Cl.: C12Q 1/6883

(54) **MICRORNAS AS PREDICTIVE BIOMARKERS OF IMMUNE RECONSTITUTION INFLAMMATORY SYNDROME**

(71) Applicant: Institut Pasteur, 75724 Paris Cédex 15 (FR)
(72) Inventor: SCOTT, Daniel, 75014 PARIS (FR); PEAN, Polidy, BP983 PHNOM PENH (KH); BORAND, Laurence, BP 983 PHNOM PENH (KH); DIM, Bunnet, BP 983 PHNOM PENH (KH); MENG, Ratana, BP 983 PHNOM PENH (KH); MADEC, Yoann, 75015 PARIS (FR)
(74) Representative: Plasseraud IP

(57) **Abstract**

The invention relates to microRNAs (miRNAs) which are predictive biomarkers of Immune reconstitution inflammatory syndrome (IRIS) and to their use for predicting and monitoring IRIS in HIV-infected subjects.

## Description

### FIELD OF THE INVENTION

The invention pertains to the field of inflammatory and immune diseases. The invention relates to microRNAs (miRNAs) which are predictive biomarkers of Immune reconstitution inflammatory syndrome (IRIS) and to their use for predicting and monitoring IRIS in HIV-infected individuals.

### BACKGROUND OF THE INVENTION

Immune reconstitution inflammatory syndrome (IRIS, or immune reconstitution disease) is a clinical entity characterized by an excessive and dysregulated inflammatory response to a preexisting antigen or pathogen which leads to a paradoxical deterioration in clinical status after initiation of antiretroviral therapy (ART) in human immunodeficiency virus (HIV) infected individuals. IRIS has also been observed in non-HIV immunocompromised patients including solid transplant organ recipients, females in postpartum period, neutropenic patients, patients on tumor necrosis factor antagonists as well as other infectious and non-infectious conditions (Sun HY et al., Curr. Opin. Infect. Dis., 2009, 4, 394-402). IRIS may present in two different ways: (1) the paradoxical worsening of symptoms of a known disease, either at a new body site or at the original body site, or (2) the unmasking of an occult opportunistic infection, in which disease that was not clinically apparent prior to ART manifests during ART. The most important co-infections implicated in the onset of IRIS in HIV-infected individuals include bacteria (*Mycobacterium tuberculosis,* other *mycobacteria* including *Avium complex, Treponema pallidum, Bartonella Quintana, Rhodococcus pneumonia*), parasites (*Toxoplama gondii, Leishmania spp., Strongyloides stercoralis, Schistosoma mansoni),* fungi (*Crytococcus neoformans, Pneumocystis jirovecii)* and viruses (Hepatitis B and C, Cytomegalovirus, Herpes virus family including Epstein-Barr virus and varicella-zoster virus, Progressive multifocal leukoencephalopathy (JC virus, JVC); Review in Vinhaes et al., Life, 2021, 11, 65, 2-26; doi.org/10.3390/life 11010065).

IRIS usually occurs in the first six months of treatment of HIV/AIDS patients. The spectrum of clinical events is diverse, with incidence ranging from 10% to 23% of all individuals who started ART and from 8% to 43% of all individuals with an existing opportunistic infection (Haddow et al., Clinical Infectious Diseases, 2009; 49:1424-32). IRIS can lead to poor adherence and compliance with highly active antiretroviral therapy (HAART) in HIV/AIDS patients. It can also increase the risk of drug resistance with HAART, worsen HIV progression to AIDS, and decrease the quality of life in HIV infected population. Overall, the IRIS has been associated with significant morbidity and mortality in HIV/AIDS patients.

Diagnosis of IRIS is difficult because of its heterogeneous clinical presentation. There is no clinical laboratory test for the diagnosis of IRIS.

MicroRNAs (miRNAs) are small (19 to 25 nt) single-stranded non-coding RNA molecules that repress gene expression and are known to be dysregulated in numerous diseases including cancer, inflammatory, infectious, metabolic and auto-immune diseases. In various diseases, miRNA expression profiles were found clinically relevant for the diagnosis, prediction, prognosis, stratification, and monitoring of therapy. Circulating miRNAs have been identified in blood serum as well as other biological fluids, where they can exist cell-free in association with the RISC complex or within small lipid vesicles such as exosomes and microvesicles. There has been significant interest in utilizing extracellular miRNAs as biomarkers because of their greater stability and predictive value than mRNA (Review in M. Alexander et al., Bioessays, 2015, 37, 1005-1015). miRNAs may be detected by conventional approaches such as real-time quantitative polymerase chain reaction (qPCR) and next generation sequencing (NGS) which are time consuming and require special equipment that are not available in all clinical laboratories, especially in the South. Alternatively, miRNA may be quantified by flow cytometry using encoded gel particle assay system which allows direct miRNA quantification from small volumes of biofluid without need for RNA purification, and with assay readout on a standard flow-cytometer (Chapin et al., Angew Chem. Int. Ed. Engl., 2011, 50, 2289-2293). After the first description of the assay, the technique evolved and improvements have been made.

The role of miRNAs is not completely defined in HIV and Tuberculosis (HIV-TB) coinfection disease. Host miRNAs target several HIV genes and consequently affect viral replication and viral control (Ghafouri-fard et al., International Immunopharmacology, 2022, 103, 108460, doi: 10.1016/j.intimp.2021.108460). In the other hand, several miRNAs in mycobacterium tuberculosis infected individuals are characterized and proposed as biomarkers of this disease (Review in Kundu et al., Front. Immunol., 2021, 12:687962, doi: 10.3389/fimmu.2021.68792). Common biomarkers associated with tuberculosis and HIV infection in co-infected patients have not been yet identified.

There is a real need for the identification of biomarkers in IRIS patients, in particular in the south countries having a high prevalence of these diseases.

### SUMMARY OF THE INVENTION

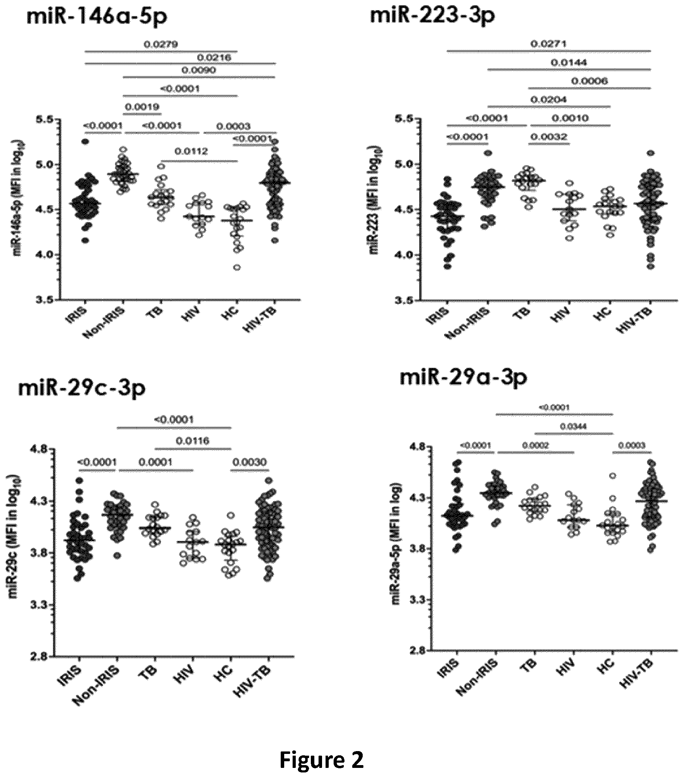

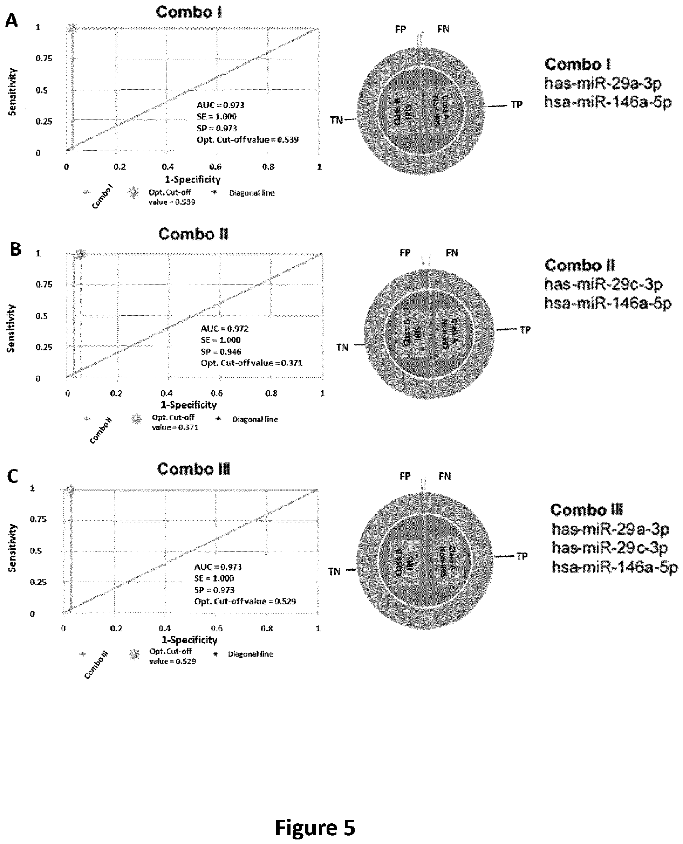

The inventors have shown that expression levels of specific circulating miRNAs that are dysregulated in HIV and tuberculosis (TB) infected subjects are significantly decreased in the subjects who developed immune reconstitution inflammatory syndrome (IRIS) after initiation of antiretroviral therapy (ART) compared to the subjects who did not develop IRIS after initiation of ART. Surprisingly, these changes in expression levels were shown in plasma samples derived from subjects before any antiretroviral and antituberculosis therapies. Thus, the risk of developing IRIS can be predicted with precision even before therapy. Four miRNAs are highly significant: hsa-miR-223-3p, hsa-miR-29a-3p, hsa-miR-29c-3p, and hsa-miR146a-5p (Tables 2, 4, 5; Figures 2 to 4). Furthermore, combinatorial analysis showed that the prediction of IRIS is improved by using the combinations of the two miRNAs hsa-miR-29c-3p/hsa-miR-146a-5p or hsa-miR-29a-3p/hsa-miR-146a-5p; or the three miRNAs hsa-miR-29c-3p/hsa-miR-29a-3p/hsa-miR-146a-5p or hsa-miR-29a-3p/hsa-miR-146a-5p/hsa-miR-223-3p (Table 6; Figure 5). The four selected miRNAs are reliable biomarkers with high specificity and validity, for predicting the occurrence of IRIS before therapy and monitoring IRIS in HIV-infected patients co-infected with tuberculosis. In fact, IRIS is an inflammatory syndrome common to several co-infections during HIV infection. If the initial infections are different, the pathophysiology of the response have similar pathways to induce inflammation. Therefore, it is reasonable to expect that miRNA implicated in regulation of the signalling described here for HIV-TB co-infection would be the same in other HIV co-infections in which IRIS is observed. At least for these reasons, these miRNA biomarkers could be used to predict IRIS in HIV-infected subjects having other co-infections.

Therefore, the invention relates to a method of determining an individual at risk of immune reconstitution inflammatory syndrome (IRIS), comprising:
- determining in a sample obtained from the individual the level of at least one microRNA (miRNA) selected from the group consisting of: hsa-miR-223-3p, hsa-miR-29a-3p, hsa-miR-29c-3p, and hsa-miR146a-5p,
wherein a reduced level of the at least one miRNA compared to a reference level indicates that the individual is at risk of developing IRIS.

In some embodiments, the individual is infected with human immune deficiency virus (HIV). In some preferred embodiments, the individual is co-infected with another pathogen; preferably chosen from Mycobacterium tuberculosis and other mycobacteria in particular of Mycobacterium avium complex, Treponema pallidum, Bartonella Quintana, Rhodococcus pneumonia, Toxoplama gondii, Leishmania spp., Strongyloides stercoralis, Schistosoma mansoni, Crytococcus neoformans, Pneumocystis jirovecii, Hepatitis B and C, Cytomegalovirus, Herpes virus family including Epstein-Barr virus and varicella-zoster virus, JC virus and JVC, more preferably Mycobacterium tuberculosis.

In some embodiments, the sample is obtained prior to treatment of the individual; preferably, prior to initiation of antiretroviral therapy; more preferably prior to initiation of antiretroviral therapy and anti-co-infection disease therapy, in particular anti-tuberculosis therapy.

In some embodiments, the method comprises determining the levels of at least two of said miRNAs, preferably at least three of said miRNAs. In some preferred embodiments, the method comprises determining the levels of the two miRNAs hsa-miR-29c-3p and hsa-miR-146a-5p or hsa-miR-29a-3p and hsa-miR-146a-5p; or the three miRNAs hsa-miR-29c-3p, hsa-miR-29a-3p and hsa-miR-146a-5p or hsa-miR-223-3p, hsa-miR-29a-3p and hsa-miR-146a-5p.

In some embodiments, the sample is a biological fluid, preferably plasma, serum or cerebrospinal fluid.

In some embodiments, the method further comprises classifying the subject as at risk of IRIS if the level of said at least one miRNA is below a reference level for the miRNA and not at risk of IRIS if the level of said at least one miRNA is equal or above the reference level for the miRNA.

In some embodiments, the level of the at least one miRNA is determined using a method selected from the group consisting of: nucleic acid sequencing, nucleic acid amplification, nucleic acid hybridization and combination thereof. In some preferred embodiments, the level of the at least one miRNA is determined using hydrogel-based assay or quantitative reverse transcription polymerase chain reaction (RT-qPCR) assay.

Another aspect of the invention relates to the *in vitro* use of at least one miRNA selected from: hsa-miR-223-3p, hsa-miR-29a-3p, hsa-miR-29c-3p, and hsa-miR146a-5p, or a combination thereof as a biomarker for predicting IRIS in a subject's sample or monitoring IRIS in a subject in need thereof. In some embodiments of the use, the combination comprises at least two of said miRNAs, preferably at least three of said miRNAs. In some preferred embodiments of the use, the combination consists of: the two miRNAs hsa-miR-29c-3p and hsa-miR-146a-5p or hsa-miR-29a-3p and hsa-miR-146a-5p; or the three miRNAs hsa-miR-29c-3p, hsa-miR-29a-3p and hsa-miR-146a-5p or hsa-miR-223-3p, hsa-miR-29a-3p and hsa-miR-146a-5p. In some embodiments of the use, the subject is infected with (HIV); preferably the subject is co-infected with another pathogen, in particular Mycobacterium tuberculosis. In some embodiments of the use, the sample is a biological fluid, preferably plasma or serum obtained. In some embodiments of the use, the sample is prior to treatment of the subject; preferably, prior to initiation of antiretroviral therapy.

### DETAILED DESCRIPTION OF THE INVENTION

The invention provides miRNAs and miRNA signatures which are predictive biomarkers of immune reconstitution inflammatory syndrome (IRIS) and their various uses and methods thereof for predicting the occurrence of IRIS before therapy and monitoring IRIS in HIV-infected subjects, in particular HIV-infected subjects having a co-infection.

As used herein, "biomarker" refers to a distinctive biological or biologically derived indicator of a process, event or condition. Biomarker includes "molecular marker" which refers to a specific gene or gene product (RNA or protein).

The term "immune reconstitution inflammatory syndrome" (IRIS) describes a collection of inflammatory disorders associated with paradoxical worsening of preexisting infectious processes following the initiation of antiretroviral therapy (ART) in HIV-infected individuals (DeSimone et al., Ann. Intern. Med., 2000, 133, 447-54; French et al., HIV Med., 2000, 1, 107-15; Shelburne et al., Medicine (Baltimore), 2002, 81, 213-27; Michelet et al., AIDS, 1998, 12, 1815-22; Hirsch et al., Clin. Infect. Dis., 2004, 38, 1159-66; Shelburne et al., J. Antimicrob. Chemother., 2006, 57, 167-70) Preexisting infections in individuals with IRIS may have been previously diagnosed and treated or they may be subclinical and unmasked by the host's regained capacity to mount an inflammatory response (French et al., Clin. Infect. Dis., 2009, 48, 101-7).

As used herein, the term "subject" or "individual" refers to a human. The subject may or may not be affected by a disease. A "patient" refers to a subject affected by a disease.

As used herein, the term "microRNA", "miRNA" or "miR" designates a non-coding RNA molecule of between 17 and 25 nucleotides which hybridizes to and regulates the expression of coding messenger RNAs. The term "miRNA molecule" refers to any nucleic acid molecule representing the miRNA including natural miRNA molecules, i.e. the mature miRNA, pre-miRNA, pri-miRNA. "miR precursor", "pre-miRNA" or "pre-miR" designates a non-coding RNA having a hairpin structure, which contains miRNA. A pre-miRNA is the product of cleavage of a primary mi-RNA transcript, or "pri-miR" by the double-stranded RNA-specific ribonuclease known as Drosha. "hsa-miR" refers to human microRNA.

The term "level" as used herein encompasses the absolute amount of a biomarker, the relative amount or concentration of the said biomarker as well as any value or parameter which correlates thereto or can be derived therefrom. Such values or parameters comprise intensity signal values from all specific physical or chemical properties obtained from the said biomarker by direct or indirect measurements. Direct measurements include for example intensity values in mass spectra or NMR spectra. Indirect measurements include for example intensity signals obtained from specifically bound ligands such as nucleic acid probes.

As used herein "level of miRNA" refers to "expression level of miRNA".

As used herein "miRNA signature" refers to a specific microRNA expression profiles representing potent biomarkers for predicting the occurrence of a disease.

The term "treating" or "treatment", as used herein, means reversing, alleviating, inhibiting the progress of, or preventing the disorder or condition to which such term applies, or reversing, alleviating, inhibiting the progress of, or preventing one or more symptoms of the disorder or condition to which such term applies.

As used herein, "drug" or "therapeutic agent" refers to a compound or agent that provides a desired biological or pharmacological effect when administered to a human or animal, particularly results in an intended therapeutic effect or response on the body to treat or prevent conditions or diseases. Therapeutic agents include any suitable biologically-active chemical compound or biologically derived component.

"a", "an", and "the" include plural referents, unless the context clearly indicates otherwise. As such, the term "a" (or "an"), "one or more" or "at least one" can be used interchangeably herein; unless specified otherwise, "or" means "and/or".

One aspect of the invention relates to a method of determining a subject's risk of immune reconstitution inflammatory syndrome (IRIS), comprising:
- determining in a sample obtained from the subject the level of at least one microRNA (miRNA) selected from the group consisting of: hsa-miR-223-3p, hsa-miR-29a-3p, hsa- hsa-miR-29c-3p, and hsa-miR-146a-5p,
wherein a reduced level of the at least one miRNA indicates that the subject is at risk of developing IRIS.

These miRNAs have shown significant reduced levels in subjects who are at risk of developing IRIS compared to subjects who are not at risk of developing IRIS. Therefore, these miRNAs represent a miRNA signature useful as biomarkers for predicting and monitoring IRIS. The determined level is compared to a predetermined level which is used as reference level and allows to predict whether a subject is at risk of occurrence of IRIS. A determined level below the reference level is indicative of the subject being at risk of developing IRIS, while a determined level equal or above a reference level is indicative of the subject not being at risk of IRIS.

In some embodiments, the method of the invention comprises:
a) determining in a sample obtained from the subject the level of at least one microRNA (miRNA) selected from the group consisting of: hsa-miR-223-3p, hsa-miR-29a-3p, hsa-miR-29c-3p, and hsa-miR146a-5p,
b) comparing said level with a reference level for said at least one miRNA,
wherein a reduced level of the at least one miRNA compared to the reference level indicates that the subject is at risk of developing IRIS.

The reference level may be the level of the miRNA in a reference sample tested simultaneously, wherein the reference sample is a sample comprising a known amount of miRNA. The reference sample may be a sample from a subject not a risk of IRIS or a pool of samples from such subjects. Alternatively, the reference is a reference value. The reference value may be a concentration or a range of concentrations of the miRNA. A reference value refers to a value established by statistical analysis of values obtained from representative panels of individuals according to standard statistical methods that are well-known in the art and disclosed in the examples.

The reference value can for example be obtained by measuring miRNA level at baseline in samples from a panel of subjects known to be at risk of IRIS (subjects who developed IRIS after initiation of ART) and a panel of subjects known not to be at risk of IRIS (subjects who did not develop IRIS after initiation of ART) as disclosed in the examples. Based on these results, a cut-off may be obtained that shows the relationship between the level(s) detected and subjects at risk. The cut-off can thereby be used to determine the level of the one or more biomarkers which corresponds to an increased risk of developing IRIS. A cut-off value that can discriminate subjects at risk and not at risk of IRIS is determined. The chosen reference level may be changed if desired to give a different sensitivity or specificity as known in the art. Cut-off values for each of the miRNA which allow to differentiate individuals at risk and not at risk of IRIS with high specificity and sensitivity are shown in Table 4.

The performance of the method may be assessed by its specificity and sensitivity. Sensitivity (SE) refers to the proportion of actual positives which are correctly identified. SE = TP/(TP+FN), where TP is the number of true positives and FN the number of false negatives. Specificity (SP) refers to the proportion of actual negatives which are correctly identified. SP= TN/TN+FP, where TN is the number of true negatives and FP the number of false positives. The relationship between both sensitivity and specificity can be assessed by the ROC curve as disclosed in the examples. This graphical representation helps to decide the optimal threshold or cut-off for a biomarker candidate.

In preferred embodiments, the invention relates to a method with a high specificity, such as at least 70%, 80%, 90%, 95% or 97% specificity. In preferred embodiments, the invention relates to a method with a high sensitivity, such as at least 70%, 80%, 90%, 95%, 97% or 100% sensitivity.

In preferred embodiments, the method comprises determining the levels of at least two of said miRNAs, preferably at least three of said miRNAs. The combination of several miRNAs increases the sensitivity and specificity of the method as shown in the examples. In some more preferred embodiments, the method comprises determining the levels of hsa-miR-29c-3p and hsa-miR-146a-5p; hsa-miR-29a-3p and hsa-miR-146a-5p; hsa-miR-29c-3p, hsa-miR-29a-3p and hsa-miR-146a-5p; or hsa-miR-223-3p, hsa-miR-29a-3p and hsa-miR-146a-5p. These specific combinations provide optimal results with a specificity of at least 95 % and a sensitivity of 100 % (Table 5 and Figure 5).

**Table 1: miRNA biomarkers**

| MicroRNA | Sequence | miRBase accession number |
|---|---|---|
| hsa-miR-223-3p | UGUCAGUUUGUCAAAUACCCCA (SEQ ID NO : 1) | MIMA T0000280 |
| hsa-miR-29a-3p | UAGCACCAUCUGAAAUCGGUUA (SEQ ID NO : 2) | MIMA T0000086 |
| hsa-miR-29c-3p | UAGCACCAUUUGAAAUCGGUUA (SEQ ID NO : 3) | MIMA T0000681 |
| hsa-miR-146a-5p | UGAGAACUGAAUUCCAUGGGUU (SEQ ID NO : 4) | MIMA T0000449 |

In some particular embodiments, the subject is infected with human immune deficiency virus (HIV). The subject has been tested positive for HIV infection using standard laboratory tests for HIV diagnostics that are well-known in the art and disclosed in the examples. The subject may be co-infected with another pathogen such as bacteria, parasite, fungus, or virus, in particular an opportunistic pathogen. In some embodiments, the individual is co-infected with HIV and a bacteria, in particular chosen from *Mycobacterium tuberculosis* (tuberculosis) and other *mycobacteria*, in particular *Mycobacterium avium complex, Treponema pallidum* (treponemosis), *Bartonella Quintana* and *Rhodococcus (equi) pneumonia*. In some embodiments, the individual is co-infected with HIV and a parasite, in particular chosen from *Toxoplama gondii* (toxoplasmosis), *Leishmania spp.* (leishmaniosis), *Strongyloides stercoralis* (strongyloidiasis) and *Schistosoma mansoni* (schistosomiasis). In some embodiments, the individual is co-infected with HIV and a fungus, in particular chosen from *Crytococcus neoformans* (cryptococcosis) or *Pneumocystis jirovecii* (pneumocystosis). In some embodiments, the individual is co-infected with HIV and another virus, in particular chosen from Hepatitis B and C, Cytomegalovirus, Herpes virus family including Epstein-Barr virus and varicella-zoster virus; JC virus and JVC (Progressive multifocal leukoencephalopathy). In some preferred embodiment, the subject is co-infected with HIV and Mycobacterium tuberculosis (M. tuberculosis). In some embodiments, the HIV-infected subject has not been diagnosed for another pathogen infection. In some embodiments, the subject has been diagnosed for another pathogen infection. The subject may have been tested positive for another pathogen infection, in particular M. tuberculosis infection. Standard laboratory tests for diagnostics of pathogen infections, in particular M. tuberculosis infection are well-known in the art and disclosed in the examples.

The risk of occurrence of IRIS is advantageously predicted early in the subject, at an early time point of disease control. The disease may be a co-infection disease such as HIV co-infection disease, in particular HIV-tuberculosis (HIV-TB) co-infection disease. The method may be performed at the time or shortly after the diagnosis of the disease affecting the subject. The method is performed before initiation of disease treatment in the subject. In particular, the prediction method may be performed at the time or shortly after the diagnosis of HIV infection, and eventually co-infection disease such as tuberculosis in the subject. In some preferred embodiments, the method is performed before initiation of antiretroviral therapy (ART). Antiretroviral therapy includes combination antiretroviral therapy (cART) and highly active antiretroviral therapy (HAART). In some particular embodiments, the method is performed prior initiation of antiretroviral and anti-co-infection disease therapies, in particular antiretroviral and anti-tuberculosis therapies. Using the method of this invention, it can be predicted whether IRIS will occur within 45 days after diagnosis of HIV infection or co-infection disease in the subject.

A sample or biologic sample refers to any material obtained or derived from living or dead individual that may contain miRNA. A biological sample may be any tissue, body fluid or stool. Non-limiting examples of body fluids include whole-blood, serum, plasma, lymph, urine, cerebrospinal fluid (CSF), and mucosal secretions, such as with no limitations oral and respiratory tract secretions (sputa, saliva and the like). Samples include swabs, aspirate, wash or lavage. Samples include direct samples (without nucleic acid isolation) and processed samples that have been treated to disrupt tissue or cell structure, thus releasing intracellular components into a solution which may further contain reagents (buffers, salts, detergents, enzymes and the like) which are used to prepare, using standard methods, a biological sample for analysis. In particular, processed samples include samples that have been treated by standard methods used for the isolation and purification of nucleic acids from biological samples. In some embodiments, the sample is a direct sample. In some other embodiments, the sample has been treated to isolate nucleic acids. For example, peripheral blood is collected in anticoagulant tubes for obtaining plasma. All tubes are processed within 1 h of collection. Samples are centrifuged at 2000 g for 10 min at room temperature to isolate plasma. Isolated plasma are transferred to RNAse- and DNAse-free tubes and stored at -80°C until required for further analysis.

In some embodiments, the sample is a biological fluid, in particular blood-sample such as serum or plasma or cerebrospinal fluid (CSF); preferably blood sample such as serum or plasma. A part of circulating mi-RNAs in cell-free biological fluids such as serum or plasma are packed in membrane-coated extracellular vesicles (EV) which are secreted by cells. EV refers to 30-1000 nm particles coated by a double-membrane layer, including exosomes, microvesicles and apototic bodies (Bryzgunova et al., Diagnostics, 2021, 11, 865). miRNAs may be extracted from extracellular vesicles such as exosomes by methods that are well-known in the art and disclosed in the examples. In some embodiments, the method comprises determining the level of the at least one miRNA in exosomes derived from a biological fluid, in particular plasma-derived exosomes.

Any suitable method for detecting and quantifying miRNA in a biological sample may be used for determining the level of the at least one miRNA in the biological sample from a subject according to the invention. Non-limit examples include: spectrophotometric methods; Northern Blotting; Microarray; in situ hybridization (ISH); Sequencing such as next generation sequencing (NGS) and Single-molecule sequencing; micro and nanosensor-based electrochemical, electrical, mechanical or optical detection, and Nucleic acid amplification techniques.

Various assays based on these methods are commercially available such as with no limitation: bead-based quantification (Illumina bead-arrays), hydrogel-based quantification (Firefly^{™}; FirePlex^{™}), micro-array technology such as chip arrays (Affymetrix, Agilent), NanoString nCounter expression system.

Point-of-care (POC) systems have also been developed for miRNA detection. These techniques include a number of isothermal amplification techniques such as branched rolling circle amplification (BRCA), exponential amplification reaction (EXPAR), hybridization chain reaction (HCR), catalytic hairpin assembly (CHA), strand-displacement amplification (SDA), duplex-specific nuclease signal amplification (DSNSA) and loop-mediated isothermal amplification (LAMP). Lateral flow assay-based devices including lateral flow nucleic acid biosensors are convenient systems used for miRNA detection. Other systems include Nano beads and electrochemical, colorimetric and microfluidic chip-based systems; oligonucleotide template reactions; pH responsive miRNA amplification method (Review in Saini et al. Egyptian Journal of Medical Human genetics, 2021,22, 4; Tribolet et al., frontiers in Microbiology, 2020, 11, doi:10.3389).

In some embodiments, miRNA present in the sample is detected by nucleic acid amplification, nucleic acid hybridization, nucleic acid sequencing assay or a combination thereof.

In particular embodiments, the miRNAs of the invention are detected using a nucleic acid probe that hybridizes with said miRNA and measuring the hybridization signal.

In particular embodiments, the level of the miRNA is determined by polymerase chain reaction (PCR). PCR methods are well-known in the art and widely used and include in particular reverse transcription-PCR (RT-PCR), quantitative PCR (Q-PCR) in particular real time Q-PCR, RT-qPCR, droplet digital PCR (ddPCR), PCR-HM (High Resolution DNA Melting, PCR coupled to ligase detection reaction based on fluorescent microspheres (Luminex^{®} microspheres), multiplex PCR, and others.

In RT-PCR, the miRNA is subjected to a reverse transcription reaction with a reverse primer before amplification. PCR amplification uses a pair of forward primer (sense primer) and reverse primer (anti-sense) specific for the miRNA according to the invention, and preferably also an oligonucleotide probe specific for the miRNA for detecting any amplified product. In the PCR methods useful in the present invention, the primers are usually based on the mature miRNA molecule but may include modifications to optimize hybridization. The reverse transcription (RT) and amplification (PCR) reactions may be performed, either in one step or in two steps. The one step reaction is performed using the reverse transcription primer as reverse primer for the amplification reaction and the same reaction mixture comprising the DNA polymerase and RT enzymes. For the two-step reaction, different primers and reaction mixtures are used for the reverse transcription and amplification reactions.

In some preferred embodiments, the levels of miRNAs are determined by RT-qPCR (or qRT-PCR). This approach involves reverse transcription (RT), PCR amplification and fluorescent detection of PCR products. Methods of determining the levels of miRNAs by qRT-PCR are known in the art. Because miRNAs are short, poly(A) tailing of miRNA 3'-ends or use of miRNA-specific-stem-loop primers enable subsequent RT. During amplification, PCR products can be monitored for example through incorporation of a fluorescent dye such as SYBR Green Dye, into double-stranded DNA or release of a fluorophore from a degraded Taqman probe. qRT-PCR methods may determine absolute level of expression of miRNA. Alternatively, qRT-PCR methods may determine the relative quantity of a miRNA. The relative quantity of a miRNA may be determined by normalizing the level of the miRNA to the level of one or more internal standard nucleic acid sequences. In general, such internal standard nucleic acid sequences should have a constant level in the analyzed sample (blood or serum sample). Examples of internal standard nucleic acid include mRNAs (GADPH, beta-actin), non-coding RNAs such as 5S and 18S ribosomal RNA, RNU44, RNU48, RNU6B and some miRNAs such as miR-451a, miR-16, miR-486, ath-mir167d (miRBase accession number MIMAT0000905); oan-miR-7417-5p (miRBase accession number MIMAT0028982) and cel-miR-70-3p (miRBase accession number MIMAT0000042). In some embodiments, the internal standard nucleic acid includes at least one of ath-mir167d, oan-miR-7417-5p and cel-miR-70-3p; preferably ath-mir167d, oan-miR-7417-5p and cel-miR-70-3p. Alternatively, the relative logarithmic difference between two miRNAs can be calculated to form self-normalizing miRNA pairs. Thereby, the need for a reference can be circumvented.

In some preferred embodiments, the levels of miRNAs are determined by hydrogel-based quantification, in particular using encoded gel particle assay system and flow cytometry (Firefly^{™}; Chapin et al., Angew Chem. Int. Ed. Engl., 2011, 50, 2289-2293) as shown in the examples. This method allows direct miRNA quantification from small volumes of biofluid without need for RNA purification, with assay readout on a standard flow-cytometer. Hydrogel-based multiplex miRNA assays including point-of-care systems are available (FirePlex^{™} from Abcam; Lee et al., Analytical chemistry, 2020, 92, 5750-5755). For example, target miRNA is hybridized to miRNA target sequences on hydrogel particles and incubated at 37°C for 60 min with shaking. Bound miRNA is labeled with fluorescent adaptor molecules by incubation at room temperature for 60 min with shaking. Eluted miRNA/adaptor complexes are amplified using single-step universal primer RT amplification consisting of 27 cycles of PCR amplification followed by 6 cycles of asymmetric amplification. Amplified products are rehybridized and incubated with reporter buffer at room temperature for 15 min with shaking. Following the addition of Run Buffer, miRNA particles are quantitatively detected on a flow cytometer previously standardised and then analysed using appropriate software. Amplification miRNA controls are included. The assay may be performed on 20 µL of plasma.

In some embodiments, the levels of at least two or three of the miRNAs according to the present disclosure are determined simultaneously using a multiplex assay, in particular a multiplex RT-qPCR assay or an hydrogel-based multiplex miRNA assay. In some embodiments, the multiplex assay comprises the determination of the levels of hsa-miR-29c-3p and hsa-miR-146a-5p; hsa-miR-29a-3p and hsa-miR-146a-5p; hsa-miR-29c-3p, hsa-miR-29a-3p and hsa-miR-146a-5p; or hsa-miR-223-3p, hsa-miR-29a-3p and hsa-miR-146a-5p .

In some embodiments, the method further comprises classifying the subject as at risk of IRIS if the level of said at least one miRNA is below a reference level for the miRNA and not at risk of IRIS if the level of said at least one miRNA is equal or above the reference level for the miRNA. Patient's stratification allows to control IRIS in patient at risk in order to improve patient's outcome. Patients classified as at risk of IRIS, will receive a personalized treatment against IRIS, such as corticoid therapy, thereby reducing side-effects of antiretroviral therapy and improving patient's well-being and patient's outcome.

Another aspect of the invention relates to a method of monitoring IRIS in a subject, comprising:
- determining the risk of occurrence of IRIS in the subject using the prediction method according to the present disclosure; and
- administering a treatment against IRIS if the subject is determined as at risk of developing IRIS.

In some embodiments, the treatment against IRIS is corticoid therapy. Corticoid therapy for treating IRIS is well known in the art (Meintjes et al., AIDS, 2010, 24, 2381-2390; doi.10.1097/QAD.0b013e32833dfc68; Meintjes et al., N. Engl. J. Med., 2018, 379, 1915-1925; doi:10.1056/NEJMoa1800762). For example, corticoid therapy may comprise prednisone at a dose of 40 mg per day for 14 days, then 20 mg per day for 14 days starting on the same day as ART or prednisone at a dose of 1.5 mg/kg per day for 14 days, then 0.75 mg/kg per day for 14 days starting on the same day as ART.

Another aspect of the invention relates to the *in vitro* use of at least one miRNA selected from: hsa-miR-223-3p, hsa-miR-29a-3p, hsa-miR-29c-3p, and hsa-miR146a-5p, or a combination thereof as a biomarker for predicting or monitoring IRIS in a subject in need thereof.

In some embodiments, the combination comprises at least two of said miRNAs, preferably at least three of said miRNAs. The combination of several miRNAs increases the sensitivity and specificity of IRIS prediction as shown in the examples. In some more preferred embodiments, the combination consists of: hsa-miR-29c-3p and hsa-miR-146a-5p; ; hsa-miR-29a-3p and hsa-miR-146a-5p; hsa-miR-29c-3p, hsa-miR-29a-3p and hsa-miR-146a-5p; or hsa-miR-223-3p, hsa-miR-29a-3p and hsa-miR-146a-5p. These specific combinations provide optimal results (Table 6 and Figure 5).

The subject is preferably HIV-infected, more preferably co-infected with HIV and another pathogen as disclosed herein, still more preferably co-infected with HIV and M. tuberculosis. The prediction of IRIS is preferably determined early as disclosed herein, specifically before initiation of antiretroviral therapy in the subject. The treatment against IRIS is any known treatment, preferably corticoid therapy as disclosed herein

The present invention also relates to an oligonucleotide probe or primer specific for any one of the miRNA biomarkers according to the present disclosure: hsa-miR-223-3p (SEQ ID NO: 1), hsa-miR-29a-3p (SEQ ID NO: 2), hsa-miR-29c-3p (SEQ ID NO: 3), and hsa-miR146a-5p (SEQ ID NO: 4).

Oligonucleotide refers to a polymer of 5 to 100 nucleotides, generally from a lower limit of 15 to 20 nucleotides to an upper limit of 45 to 60 nucleotides, preferably from about 15 to about 45 nucleotides, wherein the polymer comprises ribonucleotides, deoxyribonucleotides, modified nucleotides or mixtures thereof and may further include modified internucleotide linkages and/or modified 5' and/or 3' termini. Oligonucleotides are usually synthesized using any of a variety of well-known enzymatic or chemical methods.

The oligonucleotide primer or probe according to the disclosure hybridizes with miRNA target sequence that may RNA, cDNA, DNA equivalent or complement thereof. As used herein, "hybridize" refers to the ability to form a double-stranded hybrid (duplex) molecule with the target sequence. The oligonucleotide primer according to the invention is complementary to the target sequence. Complementary refers to partial or total complementarity. Preferably, the oligonucleotide is at least 80% identical, preferably at least 85%, 90%, 95% or 98% identical to the target sequence. The oligonucleotide may comprise additional sequences (not complementary to the target sequence) at its 5' end. In some embodiments, the oligonucleotide comprises at its 3'end, a sequence of at least 5, preferably 10 to 15 consecutive nucleotides which is 100% identical to the target sequence. Optionally, at least one oligonucleotide of the pair of amplification primers includes a label (detectable moiety). The reverse primer is an anti-sense primer which hybridizes to miRNA or DNA equivalent generated by nucleic acid amplification. The forward primer is a sense primer which hybridizes to the cDNA (complementary DNA) or DNA copies thereof generated by nucleic acid amplification. At least one oligonucleotide may also function as a probe and further includes a label to detect miRNA or an amplicon thereof. Alternatively, the amplicon is detected using a specific probe comprising a label.

A label is a moiety that can be detected directly or indirectly by the production of a detectable signal such as colorimetric, fluorescent, chemiluminescent, electrochemoluminescent or radioactive signal. Directly detectable labels include radioisotopes and fluorophores. Indirectly detectable labels have the ability to bind to or cleave another moiety, which itself may emit or absorb light of a particular wavelength and include for example, biotin, avidin, epitope tag such as the FLAGepitope, and enzyme tag such as horseradish peroxidase. Preferred labels include radioactive labels, fluorescent labels, chemiluminescent labels, bioluminescent labels, and epitope tags.

The oligonucleotides are designed based on miRNA sequences available in sequence databases using general principles for designing nucleic acid amplification primers and probes. Primer design may be performed using any of the various softwares.

In some embodiments, the oligonucleotide is a RT-stem-loop-primer comprising at its 3'end, a sequence of at least 5, preferably 10 to 15 or more consecutive nucleotides which is 100% complementary to the 3' end of the target sequence (SEQ ID NO: 1 to 4). In some embodiments, the RT-stem-loop-primer further comprises at its 5'end a sequence of at least 20 nucleotides, preferably 30 to 40 nucleotides or more that is not complementary to the target sequence. The RT-stem-loop primer consists of at least 30 nucleotides, preferably 30 to 50 nucleotides (30, 35, 40, 45 or 50 nucleotides). In some embodiments, the oligonucleotide is a forward primer comprising at its 3'end, a sequence of at least 5, preferably 10 to 15 or more consecutive nucleotides which is 100% identical to the 5' end of the target sequence (SEQ ID NO: 1 to 4). In some embodiments, the forward primer further comprises at its 5'end a sequence of at least 5 nucleotides, preferably 10 to 15 nucleotides or more that is not complementary to the target sequence. The forward primer consists of at least 15 nucleotides, preferably 15 to 30 nucleotides (15, 20, 25 or 30 nucleotides). The miRNA may be amplified in one step using the RT-stem-loop primer and the forward primer. Alternatively, the miRNA may be amplified in two steps, using the RT-stem-loop primer for the reverse-transcription reaction and the forward primer combined with a reverse primer for the PCR amplification. The reverse primer may be a universal primer comprising a part of the non-complementary sequence from the RT-stem-loop primer.

The present invention encompasses a combination of primers and/or probes specific for hsa-miR-29c-3p (SEQ ID NO: 3) and hsa-miR-146a-5p (SEQ ID NO: 4); for hsa-miR-29a-3p (SEQ ID NO: 2) and hsa-miR-146a-5p (SEQ ID NO: 4); for hsa-miR-29c-3p (SEQ ID NO: 3), hsa-miR-29a-3p (SEQ ID NO: 2) and hsa-miR-146a-5p (SEQ ID NO: 4); or for hsa-miR-223-3p (SEQ ID NO: 1), hsa-miR-29a-3p (SEQ ID NO: 2) and hsa-miR-146a-5p (SEQ ID NO: 4).

Another aspect of the invention relates to a kit for the prediction or monitoring of IRIS, comprising at least one specific oligonucleotide primer or probe or a combination of oligonucleotide primers and/or probes according to the present disclosure; preferably a labelled probe or combination thereof and/or a set of amplification primers including one labelled primer or a combination thereof. The kit optionally comprises reagents for the reverse transcription and amplification of the at least one miRNA and/or the detection of the amplification product. Reagents available for this purpose are well-known in the art and include the DNA polymerases and RT enzymes described above, buffers for the enzymes, detergents, enhancing agents, nucleic acid binding dyes and probes, preferably labelled probes. The components of the kits are packaged together into any of the various containers suitable for nucleic acid amplification such as plates, slides, wells, dishes, beads, particles, cups, strands, chips, strips and others. The primers and optional reagents may be included into any of the devices available for nucleic acid amplification including devices further integrating nucleic acid extraction and/or amplification product detection capacities such as microfluidic devices or other devices. The kit optionally includes instructions for performing at least one specific embodiment of the method of the invention.

The practice of the present invention will employ, unless otherwise indicated, conventional techniques which are within the skill of the art. Such techniques are explained fully in the literature.

The invention will now be exemplified with the following examples, which are not limitative, with reference to the attached drawings in which:

### FIGURE LEGENDS

**Figure 1****: Study design and methodology**
**Figure 2****: Plasma concentration of four-selected miRNAs at baseline**
   Four highly significant different plasmatic miRNA (see Table 2) between IRIS and Non-IRIS (HIV/TB co-infected individuals) were compared to controls groups (TB, Tuberculosis mono-infected individuals; HIV, Human Immunodeficiency Virus mono-infected individuals; HC, blood donors). HIV-TB group is both IRIS and Non-Iris individuals together. Results are expressed as log of MFI (mean of fluorescence). Significant p values are indicated.

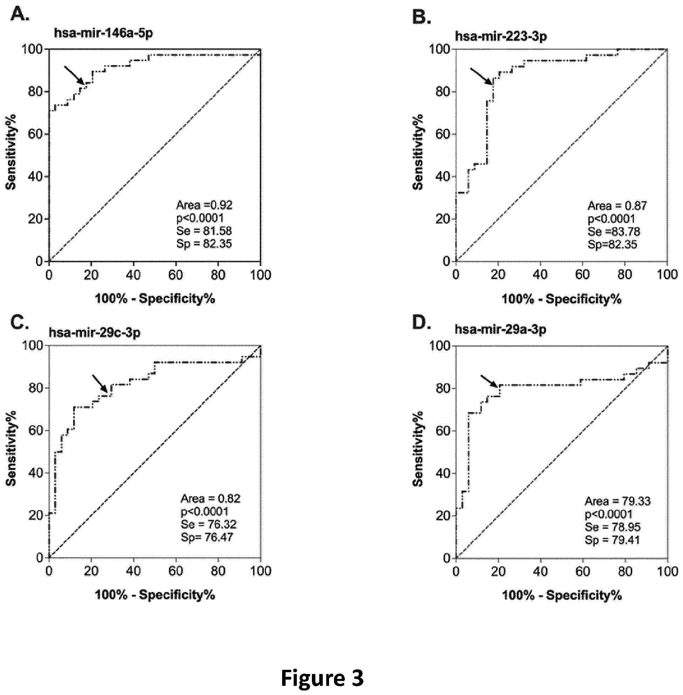

**Figure 3****: ROC curves of four-selected miRNAs between IRIS and non-IRIS patients HIV/TB co-infected individuals at baseline**
   Most significant different miRNA in plasma from HIV / Tuberculosis co-infected individuals (IRIS vs Non-IRIS) at baseline were selected for further analyses. ROC (receiver operating characteristic) curves are shown. Area under of the curves (AUC), p values, sensitivity (Se) and specificity (Sp) are indicated for each individual miRNA value.

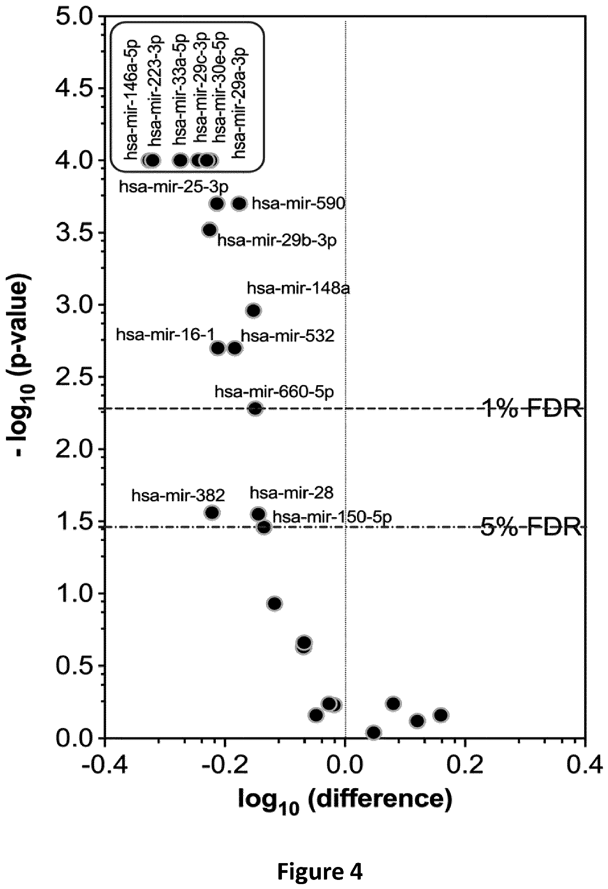

**Figure 4****: Volcano analysis of the different miRNA tested in plasma of IRIS and non-IRIS patients at baseline in HIV /TB co-infected individuals**
   Volcano plot for differential plasmatic levels of tested miRNA. The x-axis represents the log10-fold levels change in IRIS vs Non-IRIS individuals. The y-axis represents the false discovery rate (FDR) of the differential expression levels. Dot lines indicate two FDR rates. miRNA with > 4 fold change levels are in the rectangle.
**Figure 5****: Combinatorial ROC analysis of four-selected microRNAs**
   ROC curve comparison plot showing Combo I (figure 5A, hsa-miR-29a-3p, hsa-mir-146a-5p), Combo II (figure 5B, hsa-mir-29c-3p, hsa-mir-146-5p) and Combo III (figure 5c, hsa-miR-29a-3p, hsa-mir-29c-3p, hsa-mir-146a-5p). They are equally performing. Pie chart shows the fractions of false predictions (false positive, FP, and false negative, FN) as well as true predictions (true positive, TP, and true negative, TN) relative to the three Combo analysis. Combinatorial ROC analysis was done by using a free internet tool (http://combiroc.eu/) (Mazzara, S., Rossi, R., Grifantini, R. et al. CombiROC: an interactive web tool for selecting accurate marker combinations of omics data. Sci Rep 7, 45477 (2017). https://doi.org/10.1038/srep45477).

### EXAMPLES

### 1. Material and Methods

### Study design

Samples were collected retrospectively from the biobank of the CAMELIA clinical trial and CAPRI-NK study and prospectively from HIV+/TB-; HIV-/TB+ patients and from HIV-/TB- participants. The CAMELIA clinical trial (ANRS1295-CIPRA KH001-DAIDS-ES ID10425) was a prospective, randomized, multicenter, open-label, 2 arms superiority trial conducted in Cambodia, which demonstrated markedly improved survival when cART was started at 2 weeks versus 8 weeks after TB treatment in HIV/TB co-infected patients with CD4 cell ≤ 200 × 10⁶ cells/l (Blanc et al., N. Engl. J. Med., 2011, 365, 1471-1481). The early cART was also associated with a significantly increased risk of tuberculosis associated IRIS (TB-IRIS). The CAPRI NK study (ANRS12153) is an immunological study associated to the CAMELIA clinical trial in which the inventors have shown that the innate immune response plays a role in TB-IRIS and that Natural Killer cell degranulation capacity could be a predictor of TB-IRIS (Pean et al., Blood, 2012, 119, 3315-3320). The study was performed with individuals consent and in accordance with ethical regulations. The study population is described below:

### - TB and HIV co-infected patients

74 TB - HIV co-infected patients (38 TB-IRIS and 36 non TB-IRIS) were included in the project but only 72 TB - HIV co-infected patients (38 TB-IRIS and 34 non TB-IRIS) could be analyzed. The plasma from 72 TB - HIV co-infected patients were stored during the CAMELIA clinical trial and CAPRI NK sub-study. The inclusion criteria are those of the CAMELIA clinical trial, male and female subjects with a diagnosis of HIV-infection and a pulmonary or extra pulmonary TB, who have age ≥18 years, with positive HIV test results, CD4+ cell ≤ 200 × 10⁶ cells/l, positive Acid-Fast Bacilli (AFB) test on smear (sputum, lymph node, stool, CSF, pleural fluid). All plasma samples (1.5 mL) were collected before anti-retroviral (ART) and anti-TB treatment for all patients (74) and at the time of IRIS diagnosis in 22 IRIS and 22 control non-IRIS patients.

### - HIV patients (HIV+/TB-)

20 (HIV+/TB-) subjects before start of antiretroviral therapy (cART) were included in the study. The inclusion criteria are individuals whose age are ≥ 18 years with a confirmed diagnosis of HIV infection by ELISA serological testing, CD4 cell count ≤ 200 × 10⁶ cells/l and no evidence of tuberculosis infection. In order to rule out overt evidence of TB, clinical examination and whenever needed, routine TB testing were used as per national guidelines (sputum smear ± chest X-ray) in case of symptoms/clinical manifestations suggestive of TB. 5 mL blood of each patient was collected in EDTA tube before cART start.

### - Tuberculosis patients (TB+/HIV-)

20 newly active tuberculosis patients were included in the study. The inclusion criteria are individuals whose age are ≥ 18 years with no previous TB diagnosis, with confirmed AFB smear positive followed by the identification of Mycobacterium tuberculosis in culture or by MTB/RIF test. HIV-uninfected was confirmed by HIV ELISA testing. 5 mL blood of each patient was collected in EDTA tube before start of tuberculosis treatment and after week 2 and week 8 of treatment.

### - Healthy donors (HIV-/TB-)

20 healthy individuals participated in the study. The main inclusion criteria are: individuals whose ages are ≥ 18 years, negative for HIV infection confirmed by ELISA serological testing and no evidence of tuberculosis infection. In order to rule out overt evidence of TB, clinical examination and, whenever needed, routine TB testing were used as per national guidelines (sputum smear ± chest X-ray) in case of symptoms/clinical manifestations suggestive of TB. 5 mL blood of each donor was collected in EDTA tube.

### Exosome purification from plasma

After thawing and clarification of plasma by centrifugation (2000g x 20min), the plasma was centrifuged at 10000g x 20min to remove all the debris. The plasma exosome was purified from plasma by size exclusion chromatography column (qEVoriginal column , Izon Science, France). Next, the purified exosome fraction was concentrated by using Amicon Ultra-2 10K Centrifugal filter device. The exosomes in the pellet of the tube were resuspended in PBS. No further purification is needed. The fraction of concentrated exosome was used for the miRNA Flow technique.

### microRNA Profiling with FirePlex^{™} miRSelect

Raw plasma and purified exosome were used for microRNA detection. The plasma and purified exosome were digested for 45min at 60°C before hybridization. The miRSelect assay (abeam) was performed in a 96-well filter plate, allowing the simultaneous analysis of up to 96 samples and up to 68 miRNA per sample.

The following steps were done:
**1.** Addition of FirePlex particles, hybridization buffer, and samples (plasma, purified exosomes) to assay wells, followed by a 60 minutes hybridization at 37°C;
**2.** Addition of labeling Buffer, followed by 60 minutes incubation at room temperature;
**3.** The labelled microRNA in the sample was eluted into a catch plate for PCR amplification using FirePlex PCR reagent kit. Thirty-two cycles PCR were performed on one standard thermocycler (Apply Biosystem thermocycler). The PCR products were added into the assay 96-well plates for re-hybridization with the FirePlex particles.
**4.** Addition of reporter, followed by 15 minutes incubation at room temperature;
**5.** Addition of run Buffer, followed by the acquisition in standard flow cytometer.

The samples were analyzed in Fireplex Analysis Workbench software (Fireplex Analysis Workbench version 2.0.274-ext, FireCode 2.0, pickler 10 Release: Sun Mar 14 20:40:04 EDT 2021 Webstart: javaws-11.311.2.11). Positive control are particles bearing probes for a miRNA-like target, X-control, that is present in Firefly Buffer at a concentration of ~1 fmol per 25 µl. This control gives confidence that the assay was successfully implemented in every well. Blank particles bear no probe, giving a baseline level of the background fluorescence in every assay well. This signal was subtracted from those obtained by other targets in the same well. Endogenous controls are small nucleolar RNAs or microRNAs expressed at consistent levels under a variety of conditions across tissues. Three exogenous reference miroRNA were used for accuracy data normalization: ath-mir167d (miRBase accession number MIMAT0000905); oan-miR-7417-5p (miRBase accession number MIMAT0028982) and cel-miR-70-3p (miRBase accession number MIMAT0000042). A pool of normal serum was tested every time to see the variability of the test over time.

### microRNA tested

micro-RNAs were selected by their putative role in HIV and tuberculosis infection. Selection was done by search in MEDLINE, Scopus and Web of Science libraries for publications between Jan 1, 2021 and Dec 31, 2015. Twenty-six human micro-RNAs implicated in innate immunity in both diseases were selected; the micro-RNAs selected were not overlapping except one, hsa-miR-146-5p, selected as internal control. HIV: hsa-miR-29a-3p, hsa-miR-29b-1, hsa-miR-29b-2, hsa-miR-29c-3p, hsa-miR-155, hsa-miR-29b-3p, hsa-miR-33a-5p, hsa-miR-146a-5p, hsa-miR-28, hsa-miR-125b, hsa-miR-150-5p, hsa-miR-223-3p and hsa-miR-382; Tuberculosis: hsa-let-7e, hsa-miR-146a-5p, hsa-miR-148a, hsa-miR-16-1, hsa-miR-192, hsa-miR-193, hsa-miR-25-3p, hsa-miR-365-a-5p, hsa-miR-451, hsa-miR-532, hsa-miR-590-5p, hsa-miR-660-5p, hsa-miR-885-5p, hsa-miR-223-3p, hsa-miR-30e-5p. As up to 60 miRNA per well can be tested simultaneously, whole miRNA was tested in each cohort in order to better define the specificity of each miRNA.

### Statistical analysis

Expression of each sample for each miRNAs target was calculated using the geometric mean expression of microRNAs titers for microRNA selected using the geNorm algorithm. Person's correlations between the miRNAs (log-transformed) measured at baseline, Comparisons of baseline values between the groups of patients was conducted using a Kruskall-Wallis non-parametric test. When a significant difference was evidenced, two by two comparisons was conducted using a Wilcoxon non-parametric test. To correct for multiple comparisons, a FDR correction was used.

Fold change differential expression of miRNAs between groups was calculated as the ratio of normalized expression in the IRIS group and Non-IRIS group at baseline versus healthy donors.

In HIV-TB co-infected patients, Cox proportional hazard model was used to identify baseline markers associated with occurrence of IRIS. The exposure of interest in this project is baseline miRNAs (log-transformed). In the multivariate model, adjustment for already identified factors associated with occurrence of IRIS was conducted, to validate the associations identified. In HIV-TB co-infected patients, the markers were measured at baseline, and at IRIS time for those with IRIS or at a matching time (matched by study arm) for those without IRIS. The progression of the markers was compared using Wilcoxon non-parametric test for paired data. Fisher's exact test and multivariate analysis were used to compare the frequency of TB-associated clinical makers.

### - ROC analysis

The ROC curve is a plot of True Positive Rate (TPR) corresponding to sensitivity on the y-axis versus False Positive Rate (FPR) corresponding to 1 - specificity on the x-axis. TPR is defined as (number of true-positive (TP) test results) /(number of true-positive+ number of false-negative (FN) test results). FPR is defined as (number of false-positive (FP) test results) /(number of true-negative (TN) + number of false-positive (FP) test results). The area under ROC curve (AUC) reflects the ability to diagnose patients with and without the disease or condition based on the test. 0.5 suggests no discrimination. 0.7 to 0.8 acceptable. 0.8 to 0.9 excellent and more than 0.9 outstanding.

### - Stack P-value analysis

Volcano plot was constructed to visualize the significant difference between the plasmatic expression level of the set microRNA in IRIS and Non-IRIS at baseline versus the stack p-values, that computed from Wilcoxon non parametric test comparison for pair data, as described above. This analysis is to determine which of those p-values from are small enough for the corresponding comparison to be value for further statistical analysis. To decide which of those p-values are significantly small enough for further analysis, controlling the false discovery rate (FDR) approach of 1% and 5% were applied. The 5 miRNAs were selected for ROC curve analysis and ROC combinatory analysis (CombiROC).

### - CombiROC.

CombiROC approach (an interactive web-based tool, Mazzara et al., Scientific Reports, 2017, 7, 45477-; DOI: 10.1038/srep45477) was used in order to combine multiple miRNAs among the four miRNA with the best receiver operating characteristic (ROC) curves. To give an estimate of the panel performance in an unbiased manner a cross-validation (CV) procedure was introduced combined with a permutation test to over fit chance correlations.

### 2. Results

micro-RNAs were selected according to their putative role in HIV and tuberculosis infection. Twenty-six micro-RNAs implicated in innate immunity in both diseases were selected; the microRNAs were not overlapping except one, hsa-miR-146-5p, selected as internal control:
- HIV: hsa-miR-29a-3p, hsa-miR-29b-1, hsa-miR-29b-2, hsa-miR-29c-3p, hsa-miR-155, hsa-miR-29b-3p, hsa-miR-33a-5p, hsa-miR-146a-5p, hsa-miR-28, hsa-miR-125b-1, hsa-miR-150-5p, hsa-miR-223-3p and hsa-miR-382;
- Tuberculosis: hsa-let-7e, hsa-miR-146a-5p, hsa-miR-148a, hsa-miR-16-1, hsa-miR-192, hsa-miR-193, hsa-miR-25-3p, hsa-miR-365a-5p, hsa-miR-451, hsa-miR-532, hsa-miR-590-5p, hsa-miR-660-5p, hsa-miR-885-5p, hsa-miR-223-3p, hsa-miR-30e-5p.

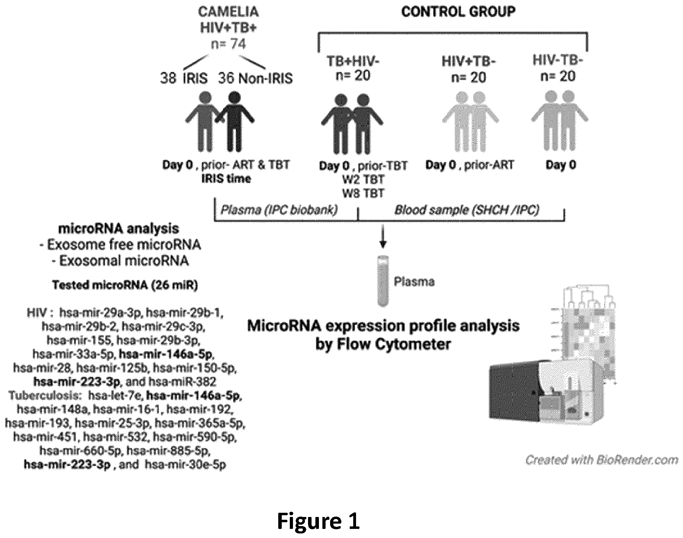

Study design and methodology are presented in **Figure 1**. Study patients were TB and HIV (HIV+/TB+) co-infected patients (n=74) including 38 IRIS and 36 non-IRIS patients from previous studies. Only 72 TB - HIV co-infected patients (38 TB-IRIS and 34 non TB-IRIS) could be analyzed for microRNA plasma levels. Plasma samples were collected before anti-retroviral (ART) and anti-TB treatments for all patients (74) and at the time of IRIS diagnosis in 22 IRIS and 22 matched control non-IRIS patients. Control groups: HIV+/TB-(n=20) before ART start; HIV-/TB+ (n=20) before anti-TB treatment and at week 2 and week 8 of anti-TB treatment; HIV-/TB- (n=20). microRNA expression profile was analysed in plasma (free miRNA) by flow cytometry.

The expression of circulating microRNAs at baseline and at IRIS onset in IRIS patients was compared to non-IRIS HIV-TB co-infected patients. 15 plasma miRNA levels at baseline were lower in IRIS vs Non-IRIS (**Table 2**). At IRIS onset, 7 plasma miRNA levels were comparable in IRIS and Non-IRIS patients (**Table 3**).

At baseline, the plasma levels of six miRNA were significantly different between IRIS and non-IRIS patients (decreased in IRIS patients); hsa-miR-223-3p, hsa-miR-29a-3p, hsa-miR-29c-3p, hsa-miR146a-5p, hsa-miR-33a-5p and hsa-miR-30e-5p (**Table 2**). Four micro-RNAs, hsa-miR-223-3p, hsa-miR-29a-3p, hsa-miR-29c-3p, and hsa-miR-146a-5p with the lowest p-value (< 0.0001) at baseline were selected for further studies.

**Table 2: Plasma miRNA expression in IRIS and non-IRIS at baseline in HIV positive individuals co-infected with Tuberculosis.**

| | miRNA | IRIS (N=38) (IQR) | Non-IRIS (N=34) (IQR) | p-value | p-value adjusted for FDR |
|---|---|---|---|---|---|
| **1** | **hsa-mir-29a-3p** | **4.12** (**4.04-4.23**) | **4.35** (**4.30-4.41**) | **9.399217e-06** | **4.229648e-05** |
| 2 | hsa-mir-29b-1 | 1.89 (1.47-2.21) | 1.77 (1.57-2.07) | 0.77 | 0.80 |
| 3 | hsa-mir-29b-2 | 2.10 (1.80-2.44) | 2.12 (1.99-2.24) | 0.59 | 0.69 |
| **4** | **hsa-mir-29c-3p** | **3.92** (**3.81-4.05**) | **4.17** (**4.06-4.20**) | **1.081775e-06** | **7.301983e-06** |
| 5 | hsa-mir-155 | 1.97 (1.72-2.29) | 2.04 (1.91-2.34) | 0.24 | 0.32 |
| **6** | **hsa-mir-29b-3p** | **3.47** (**3.37-3.67**) | **3.69** (**3.62-3.86**) | **0.00035** | **0.0009** |
| **7** | **hsa-mir-33a-5p** | **2.55** (**2.40-2.74**) | **2.82** (**2.73-2.92**) | **2.901755e-06** | **1.566948e-05** |
| **8** | **hsa-mir-146a-5p** | **4.57** (**4.47-4.75**) | **4.89** (**4.82-4.97**) | **1.358066e-11** | **3.666777e-10** |
| **9** | **hsa-mir-28** | **2.88** (**2.68-3.11**) | **3.02** (**2.92-3.16**) | **0.028** | **0.048** |
| 10 | hsa-mir-125b-1 | 1.93 (1.48-2.27) | 1.77 (1.56-2.15) | 0.69 | 0.75 |
| 11 | hsa-mir-150-5p | 2.81 (2.59-3.15) | 2.95 (2.82-3.23) | 0.035 | 0.055 |
| **12** | **hsa-mir-223-3p** | **4.43** (**4.29-4.56**) | **4.75** (**4.64-4.83**) | **8.647457e-08** | **7.782712e-07** |
| **13** | **hsa-mir-382** | **3.16** (**2.93-3.49**) | **3.38** (**3.15-3.70**) | **0.028** | **0.048** |
| 14 | hsa-let7e | 2.46 (2.41-2.84) | 2.66 (2.50-3.00) | 0.12 | 0.18 |
| **15** | **hsa-mir-148a** | **4.06** (**3.94-4.45**) | **4.21** (**4.14-4.37**) | **0.001** | **0.003** |
| **16** | **hsa-mir-16-1** | **2.24** (**2.15-2.41**) | **2.45** (**2.34-2.52**) | **0.002** | **0.004** |
| 17 | hsa-mir-192 | 3.20 (2.88-3.74) | 3.22 (3.04-3.59) | 0.58 | 0.69 |
| 18 | hsa-mir-193 | 2.15 (1.79-2.56) | 2.11 (1.94-2.40) | 0.91 | 0.91 |
| **19** | **hsa-mir-25-3p** | **4.22** (**4.13-4.38**) | **4.43 (**4**.31-4.53**) | **0.0002** | **7.449495e-04** |
| 20 | hsa-mir-365a-5p | 1.96 (1.59-2.38) | 1.88 (1.59-2.13) | 0.57 | 0.69 |
| 21 | hsa-mir-451 | 4.93 (4.61-5.31) | 5.00 (4.90-5.23) | 0.22 | 0.31 |
| **22** | **hsa-mir-532** | **2.84** (**2.60-3.02**) | **3.02** (**2.92-3.12**) | **0.002** | **0.004** |
| **23** | **hsa-mir-590-5p** | **2.81** (**2.71-2.88**) | **2.98** (**2.85-3.18**) | **0.0002** | **0.0007** |
| **24** | **hsa-mir-660-5p** | **3.38** (**3.26-3.59**) | **3.54** (**3.46-3.67**) | **0.005** | **1.028004e-02** |
| 25 | hsa-mir-885-5p | 2.71 (2.45-3.54) | 2.76 (2.52-3.08) | 0.68 | 0.75 |
| **26** | **hsa-mir-30e-5p** | **2.73** (**2.61-2.87**) | **2.96** (**2.83-3.04**) | **7.855306e-05** | **3.029904e-04** |

| | | | | | |
|---|---|---|---|---|---|
| *Comparison of the plasma levels of the 26 miRNA markers between IRIS and non-IRIS patients at baseline using Wilcoxon non-parametric test. In bold, it is indicated the difference that remained significant after controlling for multiple testing using a FDR (False Discovery rate) correction.* | | | | | |

**Table 3: Plasma miRNA expression in IRIS and non-IRIS at the time of IRIS diagnosis in HIV positive individuals co-infected with Tuberculosis.**

| **Plasma mi-RNA** | **IRIS vs NON-IRIS** | **p-value** |
|---|---|---|
| hsa-miR-150-5p | = | 0.222 |
| hsa-miR-148a | > | 0.005 |
| hsa-miR-223-3p | < | 0.008 |
| hsa-miR-29a-3p | < | 0.039 |
| hsa-miR-29b-3p | = | 0.209 |
| hsa-miR-29c-3p | < | 0.042 |
| hsa-miR-33a-5p | < | 0.054 |
| hsa-miR-146a-5p | = | 0.337 |
| hsa-miR-532 | = | 0.655 |
| hsa-miR-16-1 | = | 0.156 |
| hsa-miR-25-3p | < | 0.002 |
| hsa-miR-590-5p | = | 0.949 |
| hsa-miR-660-5p | = | 0.588 |
| hsa-miR-30e-5p | < | 0.063 |
| hsa-miR-451 | < | 0.033 |

The expression levels of the four-selected plasma microRNAs at baseline of HIV+/TB+ (IRIS and Non-IRIS) patients, HIV and TB mono-infected patients and controls is shown in **Figure 2**. It is observed that the expression levels of the four-selected plasma miRNA are significantly lower in IRIS versus Non-IRIS patients. The ROC curves (specificity and sensitivity) of the selected miRNA were then analysed to differentiate IRIS at the baseline. As shown in **Figure 3** **and Table 4**, ROC curves confirm the differences observed before.

**Table 4: ROC analysis of four-selected plasma miRNAs**

| **miRNA** | **Area under curve (AUC)^{∗}** | **p-value** | **Sensitivity (SE) %** | **Specificity (SP)%** |
|---|---|---|---|---|
| hsa-miR-146a-5p | 0.9187 (0.8522-0.9853) | < 0.0001 | 82.35 | 81.58 |
| hsa-miR-223-3p | 0.8696 (0.7837-0.9556) | < 0.0001 | 82.35 | 83.78 |
| hsa-miR-29c-3p | 0.8197 (0.7178-0.9215) | < 0.0001 | 76.47 | 76.32 |
| hsa-miR-29a-3p | 0.7933 (0.6783-0.9084) | < 0.0001 | 79.41 | 78.95 |

| | | | | |
|---|---|---|---|---|
| ^{∗} 95 % CI | | | | |

Cut-off values allowing to differentiate patients at risk of IRIS (having a level of the miRNA biomarker below the cut-off value for said biomarker) from patient not at risk of IRIS (having a level of the miRNA biomarker equal or above the cut-off value for said biomarker) were determined for the four selected miRNAs (**Table 5**).

**Table 5: Cut-off values of the 4 selected miRNAs**

| **Selected miRNA** | **Cut-off value** (**MFI in log**) | **Sensitivity^{∗}** (**SE**) **%** | **Specificity^{∗}** (**SE**) **%** | **Likelihood ratio** |
|---|---|---|---|---|
| hsa-miR-146a-5p | 4.801 | 81.58 (66.58-90.78) | 82.35 (66.49-91.65) | 4.623 |
| hsa-miR-223-3p | 4.574 | 83.78 (68.86-92.35) | 82.35 (66.49-91.65) | 4.748 |
| hsa-miR-29c-3p | 4.054 | 76.32 (60.79-87.01) | 76.47 (60.00-87.56) | 3.243 |
| hsa-miR-29a-3p | 4.287 | 78.95 (63.65-88.93) | 79.41 (63.20-89.65) | 3.835 |

| | | | | |
|---|---|---|---|---|
| ^{∗} 95 % CI | | | | |

Moreover, stack-p values with control False Discovery Rate analysis of the miRNA also confirms the utility to differentiate IRIS and Non-IRIS patients at baseline (**Figure 4**).

A Combinatorial ROC analysis of the 4 microRNAs was performed. Several combinations were tested. The best results (1 or near to) in the following parameters: Accuracy (ACC), Sensitivity (SE), Specificity (SP), lower error (ER) (near to 0) are indicated (**Table 6**).

**Table 6: Best combination of ROC curves**

| **Combination** | **Data set type** | **ACC** | **ER** | **SE** | **SP** | **AUC** | **Optimal Cut-off** |
|---|---|---|---|---|---|---|---|
| **hsa-miR-29a-3p hsa-miR-146a-5p** | **Whole cohort** | **0.986** | **0.014** | **1** | **0.973** | **0.973** | **0.539** |
| | **10-fold CV** | **0.986** | **0.014** | **1** | **0.973** | **0.973** | |
| **hsa-miR-29c-3p hsa-miR-146a-5p** | **Whole cohort** | **0.972** | **0.028** | **1** | **0.946** | **0.972** | **0.371** |
| | **10-fold CV** | **0.958** | **0.042** | **0.941** | **0.973** | **0.918** | |
| **hsa-miR-29a-3p hsa-miR-29c-3p hsa-miR-146a-5p** | **Whole cohort** | **0.986** | **0.014** | **1** | **0.973** | **0.973** | **0.529** |
| | **10-fold CV** | **0.972** | **0.028** | **1** | **0.946** | **0.973** | |
| **hsa-miR-29a-3p hsa-miR-146a-5p hsa-miR-223-3p** | **Whole cohort** | **0.986** | **0.014** | **1** | **0.973** | **0.973** | **0.366** |
| | **10-fold CV** | **0.958** | **0.042** | **0.941** | **0.973** | **0.942** | |
| hsa-miR-29c-3p hsa-miR-146a-5p hsa-miR-223-3p | Whole cohort | 0.958 | 0.042 | 1 | 0.919 | 0.971 | 0.272 |
| | 10-fold CV | 0.944 | 0.056 | 0.941 | 0.946 | 0.914 | |
| hsa-miR-29a-3p hsa-miR-29c-3p hsa-miR-146a-5p has-miR-223-3p | Whole cohort | 0.972 | 0.028 | 1 | 0.946 | 0.972 | 0.354 |
| | 10-fold CV | 0.944 | 0.056 | 0.941 | 0.946 | 0.926 | |

The combinations of the 2 microRNAs (hsa-miR-29c-3p/hsa-miR-146a-5p or hsa-miR-29a-3p/hsa-miR-146a-5p) or the 3 microRNAs (hsa-miR-29c-3p/ hsa-miR-29a-3p/hsa-miR-146a-5p or hsa-miR-29a-3p/hsa-miR-146a-5p/hsa-miR-223-3p) increases the test performance to differentiate IRIS from non-IRIS at baseline (**Table 6**, **Figure 5**).

Taken together, this study shows that HIV/TB coinfection displays a dysregulation of circulating microRNA expression. The results indicate that hsa-miR-223-3p, hsa-miR-29a-3p, hsa-miR-29c-3p, and miR146a-5p are good candidate biomarkers to predict the occurrence of IRIS in HIV co-infected patients before any treatments. Measurement of plasma hsa-miR-223-3p, hsa-miR-29a-3p, hsa-miR-29c-3p, and miR146a-5p in HIV-TB co-infected patients at baseline could be used as a predictor of IRIS. The prediction of IRIS is improved by using the combinations of the 2 microRNAs (hsa-miR-29c-3p/hsa-miR-146a-5p or hsa-miR-29a-3p/hsa-miR-146a-5p) or the 3 microRNAs (hsa-miR-29c-3p/ hsa-miR-29a-3p/hsa-miR-146a-5p or hsa-miR-29a-3p/hsa-miR-146a-5p/hsa-miR-223-3p).

## Claims

1. A method of determining a subject's risk of immune reconstitution inflammatory syndrome (IRIS), comprising:
- determining in a sample obtained from the subject the level of at least one microRNA (miRNA) selected from the group consisting of: hsa-miR-223-3p, hsa-miR-29a-3p, hsa-miR-29c-3p, and hsa-miR-146a-5p,
wherein a reduced level of the at least one miRNA compared to a reference level indicates that the subject is at risk of developing IRIS.

2. The method according to claim 1, wherein the subject is infected with human immune deficiency virus (HIV).

3. The method according to claim 2, wherein the subject is co-infected with another pathogen; preferably chosen from Mycobacterium tuberculosis and other mycobacteria in particular of Mycobacterium avium complex, Treponema pallidum, Bartonella Quintana, Rhodococcus pneumonia, Toxoplama gondii, Leishmania spp., Strongyloides stercoralis, Schistosoma mansoni, Crytococcus neoformans, Pneumocystis jirovecii, Hepatitis B and C, Cytomegalovirus, Herpes virus family including Epstein-Barr virus and varicella-zoster virus, JC virus and JVC; more preferably Mycobacterium tuberculosis.

4. The method according to any one of claims 1 to 3, wherein the sample is obtained prior to treatment of the subject.

5. The method according to claim 4, wherein the sample is obtained prior to initiation of antiretroviral therapy, preferably prior to initiation of antiretroviral therapy and anti-co-infection disease therapy, in particular anti-tuberculosis therapy.

6. The method according to any one of the preceding claims, comprising determining the levels of at least two of said miRNAs, preferably at least three of said miRNAs.

7. The method according to claim 6, comprising determining the levels of the two miRNAs hsa-miR-29c-3p and hsa-miR-146a-5p or hsa-miR-29a-3p and hsa-miR-146a-5p; or the three miRNAs hsa-miR-29c-3p, hsa-miR-29a-3p and hsa miR-146a-5p or hsa-miR-223-3p, hsa-miR-29a-3p and hsa-miR-146a-5p.

8. The method according to any one of the preceding claims, wherein said sample is a biological fluid, preferably plasma, serum or cerebrospinal fluid.

9. The method according to any one of the preceding claims, further comprising classifying the subject as at risk of IRIS if the level of said at least one miRNA is below the reference level for the miRNA and not at risk of IRIS if the level of said at least one miRNA is equal or above the reference level for the miRNA.

10. The method according to any one of the preceding claims, wherein the level of the at least one miRNA is determined using a method selected from the group consisting of: nucleic acid sequencing, nucleic acid amplification, nucleic acid hybridization and combination thereof.

11. The method according to claim 10, wherein the level of the at least one miRNA is determined using hydrogel-based assay or quantitative reverse transcription polymerase chain reaction (RT-qPCR) assay.

12. *In vitro* use of at least one miRNA selected from: hsa-miR-223-3p, hsa-miR-29a-3p, hsa-miR-29c-3p, and hsa-miR146a-5p, or a combination thereof as a biomarker for predicting IRIS in a subject's sample or monitoring IRIS in a subject in need thereof.

13. The use according to claim 12, wherein the combination comprises at least two of said miRNAs, preferably at least three of said miRNAs.

14. The use according to claim 12 or claim 13, wherein the combination consists of: the two miRNAs hsa-miR-29c-3p and hsa-miR-146a-5p or hsa-miR-29a-3p and hsa-miR-146a-5p; or the three miRNAs hsa-miR-29c-3p, hsa-miR-29a-3p and hsa-miR-146a-5p or hsa-miR-223-3p, hsa-miR-29a-3p and hsa-miR-146a-5p.

15. The use according to any one of claims 12 to 14, wherein the subject is as defined in claim 2 or 3 or the subject's sample is as defined in any one of claims 4, 5 or 8.
